# EUROPEAN PATENT SPECIFICATION

(11) **EP 3 253 069 B1**
(45) Date of publication and mention of the grant of the patent: **09.06.2021**
(21) Application number: 15879318.2
(22) Date of filing: 26.01.2015
(51) Int. Cl.: H04R 1/10

(54) **EARPHONE NOISE REDUCTION METHOD AND APPARATUS**
VERFAHREN UND VORRICHTUNG ZUM VERHINDERN VON KOPFHÖRERRAUSCHEN
PROCÉDÉ ET APPAREIL DE RÉDUCTION DU BRUIT DANS UN ÉCOUTEUR

(43) Date of publication of application: 06.12.2017
(73) Proprietor: Shenzhen Grandsun Electronic Co., Ltd., Shenzhen, Guangdong 518117 (CN)
(72) Inventor: SONG, Yanan, Shenzhen, Guangdong 518117 (CN); WU, Haiquan, Shenzhen, Guangdong 518117 (CN); SHI, Ruiwen, Shenzhen, Guangdong 518117 (CN); GENG, Xinian, Shenzhen, Guangdong 518117 (CN); DU, Guozhong, Shenzhen, Guangdong 518117 (CN); XU, Jiayun, Shenzhen, Guangdong 518117 (CN)
(74) Representative: MERH-IP Matias Erny Reichl Hoffmann Patentanwälte PartG mbB
(86) International application number: PCT/CN2015/071567
(87) International publication number: WO 2016/119106

(56) References cited:
- CN-A- 101 951 422
- CN-A- 103 024 631
- CN-A- 103 024 631
- CN-A- 103 078 997
- CN-A- 103 716 438
- US-A1- 2014 105 412
- US-A1- 2014 126 733

## Description

### FIELD OF THE INVENTION

The application relates to the technical field of wearable devices, and in particular to an earphone noise reduction method and apparatus.

### BACKGROUND OF THE INVENTION

As one of the four major internationally-recognized pollutions, noise pollution has become increasingly serious with urbanization development and technological progress. When a user plays audio through a terminal external earphone, he/she can utilize an earphone noise reduction method to reduce environmental noise and protect his/her hearing.

Earphone noise reduction methods in the prior art are achieved in two ways, the first way is blocking the noise physically using the earphones, such that the noise cannot reach the user's ears; the second way is collecting, by the earphones, the noise, reversing and superposing the noise inside the earphones so as to compensate the noise inside the earphones, such that the noise cannot be perceived by the ears.

However, the existing earphone noise reduction methods require keys or toggle switches arranged on the earphones configured to regulate the noise reduction, which is unfavorable for advanced integration of the earphones, and automatic noise reduction functions cannot be enabled or disabled in time. The reason is that the existing earphone noise reduction methods such as the first way and the second way, all utilize the keys or toggle switches on the earphones to enable or disable the noise reduction function manually. These ways generally result in inappropriate use of the noise reduction function because of the user's subjective misplay or distraction. For example, when the noise is high enough to damage the user's hearing, however, the noise reduction function is not enabled in time, which may damage the user's hearing; or when environmental noise signal cannot be perceived any more, however, the noise reduction function is still enabled. Therefore, the noise reduction function cannot be enabled or disabled in time, which incurs unnecessary power consumption and decreases cruising power of the earphones. Meanwhile, the earphone noise reduction methods in the prior art require manufacturer to arrange hardware configured for regulating noise reduction on the earphones, which decreases the integration degree of the earphones.

US2014105412A1, with title of User designed active noise cancellation (ANC) controller for headphones; which disclosed enabling headphones to perform active noise cancellation for a particular user.

CN103024631, with title of Method and device of noise reduction of earphone; which disclosed a mobile terminal obtains environment noise; the mobile terminal generates a signal with the same amplitude and the opposite phase of the noise; and the mobile terminal outputs the signal through the earphone when outputs voice frequency.

US 20140126733, with title of User Interface for ANR Headphones with Active Hear-Through; which disclosed an active noise reducing headphone has an active noise-cancelling mode and an active hear-through mode, and changes between the noise-cancelling mode and the hear-through mode based on detection of a user touching a housing of the headphone or based on a command signal received from an external device.

### SUMMARY OF THE INVENTION

The purpose of the embodiments of the present application is to provide an earphone noise reduction method, which aims to deal with issues existing in the earphone noise reduction methods in the prior art; the issues are that keys or toggle switches for regulating noise reduction are arranged on the earphones, which is unfavorable for advanced integration of the earphones, and the noise reduction function cannot be enabled or disabled in time.

The embodiments of the present application are realized as follows: the earphone noise reduction method includes:
utilizing an earphone microphone to collect noise signal of an environment where the earphone microphone is positioned;
transmitting the noise signal to a connected terminal, or transmitting a noise value corresponding to the noise signal to the connected terminal;
receiving a judgement result returned by the terminal, and enabling a noise reduction function or disabling the noise reduction function according to the judgement result.

Another purpose of the embodiments of the present application is to provide an earphone noise reduction apparatus which includes:
a noise signal collection module configured to utilize an earphone microphone to collect noise signal of an environment where the earphone microphone is positioned;
a noise signal transmitting module configured to transmit the noise signal to a connected terminal, or transmit a noise value corresponding to the noise signal to the connected terminal;
a noise reduction function module configured to receive a judgement result returned by the terminal, and enable a noise reduction function or disable the noise reduction function according to the judgement result.

In the embodiments of the present application, the judgement result returned by the terminal is received, and the noise reduction function is enabled or disabled according to the judgement result, which deals with issues existing in the earphone noise reduction methods in the prior art that keys or toggle switches for regulating noise reduction require to be arranged on the earphones, which is unfavorable for advanced integration of the earphones, and the noise reduction function cannot be enabled or disabled in time. The present application has two beneficial effects. On one hand, a manufacturer does not need to arrange a hardware switch for noise reduction adjustment on earphones, and on the other hand, automatic noise reduction is realized, and on the premise that the hardware switch on the earphones is not needed, a noise reduction function of the earphones can be automatically enabled or disabled, thereby improving the integration degree of the earphones, decreasing unnecessary power consumption and enhancing cruising power of the earphones.

### BRIEF DESCRIPTION OF THE DRAWINGS

Figure 1 is an implementation flow chart of an earphone noise reduction method provided by an embodiment of the present application;
Figure 2 is an implementation flow diagram of S 102 of the earphone noise reduction method provided by an embodiment of the present application;
Figure 3 is an implementation flow diagram of S103 of the earphone noise reduction method provided by an embodiment of the present application;
Figure 4 is a first structural block diagram of an earphone noise reduction apparatus according to an embodiment of the present application;
Figure 5 is a second structural block diagram of the earphone noise reduction apparatus according to an embodiment of the present application;
Figure 6 is a third structural block diagram of the earphone noise reduction apparatus according to an embodiment of the present application.

### DETAILED DESCRIPTION OF THE PREFERRED EMBODIMENT

In order to make the purposes, technical solutions and advantages of the present application clearer, the present application will be described in further detail with reference to the accompanying drawings and embodiments. It is to be understood that the specific embodiments described herein are merely illustrative of the application and are not intended to limit the application.

### Embodiment 1

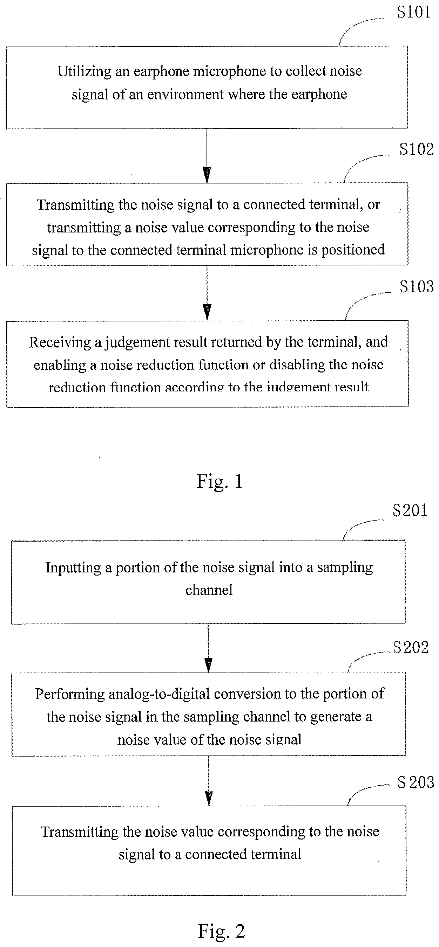

Figure 1 is an implementation flow chart of the earphone noise reduction method provided by the embodiment of the present application, which would be described in detail as below:
S101, utilizing an earphone microphone to collect noise signal of an environment where the earphone microphone is positioned;
   wherein, two implementations for the step of "utilizing an earphone microphone to collect noise signal of an environment where the earphone microphone is positioned" will be described in detail as follows:
   The first implementation:
      an earphone microphone is utilized to collect noise signal of an environment where the earphone microphone is positioned every predetermined detection interval.
      It should be understood that since noise signal of the environment does not change significantly within a period of time, power consumption of the earphones can be further saved by detecting the noise signal of the environment once every predetermined detection interval.
   The second implementation:
      When a distance between the geographic position where earphones is currently positioned and a geographic position where the noise signal of the environment where the earphone microphone was positioned was collected last time is not less than a predetermined distance, an earphone microphone is utilized to collect noise signal of the environment where the earphone microphone is positioned.

It should be understood that if the distance between the geographic position where earphones is currently positioned and the geographic position where the noise signal of the environment where the earphone microphone was positioned was collected last time exceeds the predetermined distance, the environment changes accordingly because of geographical distance. A further detection to the noise signal of the environment is performed in order to inform the user of changing situation of the signal noise intensity of the environment where the user is positioned timely.

It should be understood that since one condition may happen, in which the distance which the earphones move does not exceed the predetermined distance, whereas the moving duration is relatively long, in order to avoid that noise signal intensity changes significantly of the environment where the earphones are positioned during the long moving duration, and the noise signal cannot be collected synchronously, S101 is further specified into:
When a distance between the geographic position where earphones is currently positioned and the geographic position where the noise signal of the environment where the earphone microphone was positioned was detected last time does not exceed the predetermined distance, whereas the duration since the noise signal of the environment was detected last time by the earphones exceeds a predetermined detection interval, then the earphones also collect noise signal where the earphone microphone is positioned.

Therefore, accuracy and instantaneity of the noise signal detection of the environment where the earphone microphone is positioned is further improved.

Identically, it should be understood that earphones' native GPS function can be utilized to obtain distance from current position of the earphones to the graphical position where the noise signal of the environment where the earphones were positioned was detected last time, so as to judge whether the distance between the geographic position where earphones is currently positioned and the graphical position where the noise signal of the environment where the earphones were positioned was detected last time exceeds the predetermined distance.

S102, transmitting the noise signal to a connected terminal, or transmitting a noise value corresponding to the noise signal to the connected terminal.

Wherein the noise signal can be transmitted directly to the connected terminal, alternatively, the noise signal can be sampled to generate a noise value corresponding to the noise signal, which is then transmitted to a terminal. Wherein the terminal is a device which exchanges instructions and data with the earphones in a wired or wireless mode.

The terminal includes but not limited to smart phones, tablets, laptops, desktop computers and smart televisions.

It should be understood that the terminal can be connected with the earphones through any wired modes or any wireless modes. The wireless modes include but not limited to Bluetooth, WIFI, 3G, 4G and 5G. Wherein the earphones have no display screen and are only capable of enabling or disabling noise reduction functionally, which can neither provide users with any visualized information and suggestion, nor provide users with flexible scheme for regulating the noise reduction. By transmitting the noise signal or the noise value corresponding to the noise signal to the connected terminal, the earphones need not to judge the noise signal, which decrease the burden of a processor of the earphones for processing data; meanwhile, the earphones are capable of receiving scheme for regulating the noise reduction transmitted by the connected terminal.

S103, receiving a judgement result returned by the terminal, and enabling a noise reduction function or disabling the noise reduction function according to the judgement result.

The earphones receive the judgement result returned by the terminal through a default communication interface, and based on the judgement result, detect whether the current state of the noise reduction function is identical with the judgement result. If identical, then the current state of the noise reduction function is maintained, otherwise, the current state of the noise reduction function is changed and the noise reduction function is abled or disabled.

In the embodiment of the present application, the judgement result returned by the terminal is received, and the noise reduction function is enabled or disabled according to the judgement result, which solves the problems in the existing earphone noise reduction methods that the requirement for arranging keys or toggle switches for noise reduction adjustment on earphones is unfavorable for advanced integration of the earphones and the noise reduction function cannot be enabled or disabled in time. By replacing traditional manual noise reduction with automatic noise reduction, the earphones are not only more humanized and intelligentized, but also capable of meeting the requirement of the user for hearing health.

### Embodiment 2

Figure 2 is an implementation flow diagram of S102 of the earphone noise reduction method provided by the embodiment of the present application, which would be described in detail as below:
5201, inputting a portion of the noise signal into a sampling channel;
S202, performing analog-to-digital conversion to the portion of the noise signal in the sampling channel to generate a noise value of the noise signal;
S203, transmitting the noise value corresponding to the noise signal to a connected terminal.

In the embodiment of the present application, the noise value corresponding to the noise signal is transmitted to the connected terminal, which is convenient for receiving a judgement result generated by the terminal based on the noise signal, and enabling a noise reduction function or disabling the noise reduction function according to the judgement result.

### Embodiment 3

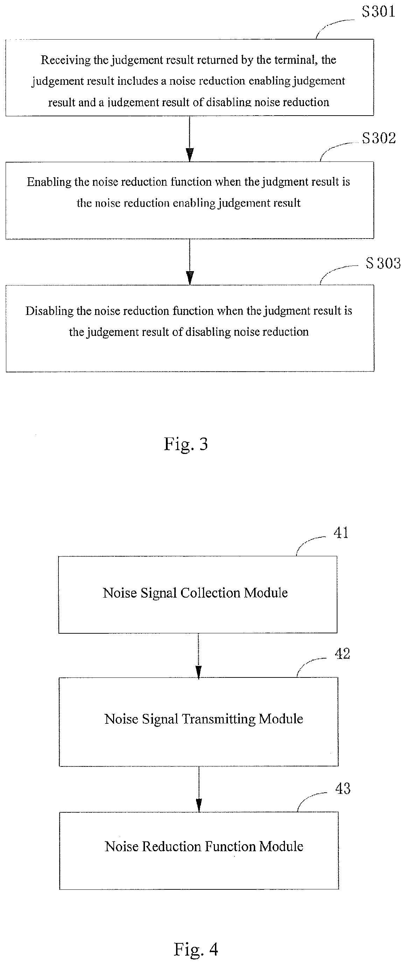

Figure 3 is an implementation flow diagram of S103 of the earphone noise reduction method provided by the embodiment of the present application, which would be described in detail as follows:
5301, receiving the judgement result returned by the terminal, the judgement result includes a judgement result of enabling noise reduction and a judgement result of disabling noise reduction;
5302, enabling the noise reduction function when the judgment result is the judgement result of enabling noise reduction;

When the judgment result is the judgement result of enabling noise reduction, whether the current state of the noise reduction function of the earphones is enabled is detected; when the current state of the noise reduction function of the earphones is not in enabled state, then the noise reduction function is adjusted to be enabled.

S303, disabling the noise reduction function when the judgment result is the judgement result of disabling noise reduction;
When the judgment result is the judgement result of disabling noise reduction, whether the current state of the noise reduction function of the earphones is disabled is detected; when the current state of the noise reduction function of the earphones is not in disabled state, then the noise reduction function is adjusted to be disabled.

In the embodiment of the present application, the noise reduction function is enabled or disabled according to the judgement result, which achieves automatic noise reduction function of the earphones, improves the integration degree of the earphones and enhances the cruising power of the earphones.

### Embodiment 4

The embodiment of the present application mainly described a preferred implementation process of the application in a practical application, which would be described in detail as follows:
there are two processing modes after the earphone microphone collect noise signal of the environment where the earphone microphone is positioned:
If an analogue noise reduction technology is used, then environment noise signal is divided into two paths: one path utilizes ADC conversion to perform numerical calculation so as to obtain a decibel value of the noise signal; the other path acts as analog signal to be input into an active noise reduction module.

If a digital noise reduction technology is used, then the environmental noise signal is directly input into the active noise reduction module; calculation is performed inside the module and the decibel value of the noise signal is returned.

The decibel value is generally transmitted to the terminal via any transmission mode. For example, the transmission modes include but not limited to Bluetooth transmission mode, WIFI transmission mode, 3G transmission mode, 4G transmission mode and 5G transmission mode.

After receiving the decibel value of the noise signal, the terminal analyzes the decibel value and returns two contents:
The first content: displaying an analysis result of current environmental noise signal on the terminal, and providing suggestion for a user about how to use the earphones.
The second content: sending corresponding instruction automatically to the earphone, and adjusting the function of the earphones to a usage state which is favorite for the current noise environment.

If the user refuses the best usage state of the earphones controlled by the terminal, the user may regulate the usage state of the earphones by adjusting the terminal or the earphones.

### Embodiment 5

The embodiment of the present application describes preferred implementation processes for three different scenes, which would be described in detail as below:
The first scene, a smartphone provides noise reduction suggestion based on the decibel value of environmental noise signal collected by the earphones, which would be described in detail as follow:
After the earphone microphone collects the environmental noise signal, the environmental noise signal is divided into two paths: one path utilizes ADC conversion to perform numerical calculation so as to obtain a decibel value of the noise signal, which is 80dB.

The obtained decibel value is transmitted to the smartphone via Bluetooth.

After receiving the decibel value, the smartphone analyzes the decibel value, and learns that noise with a decibel value of 80dB would significantly damage a human's hearing, and the volume has been overly turned up by the current user.

After the noise reduction function is enabled, current volume level used by the user would be overly high; therefore, the smartphone would display analysis result and application suggestion. The analysis result and application suggestion are as follows:
The smartphone suggests the user to enable the earphone noise reduction function, and turn down the volume level of the earphones or phone so as to protect the hearing.

Secondary, the smartphone transmits instruction of enabling the active noise reduction function to the earphones automatically, and turns down the volume level of the earphones.

The second scene, a tablet provides noise reduction suggestion based on the decibel value of environmental noise signal collected by the earphones, which would be described as below:
After the earphone microphone collects the environmental noise signal, the environmental noise signal is divided into two paths: one path utilizes ADC conversion to perform numerical calculation so as to obtain a decibel value of the noise signal, which is 20dB.

The obtained decibel value is transmitted to the tablet via WIFI.

After receiving the decibel value, the tablet analyzes the decibel value, and learns that noise with a decibel value of 20dB would not damage a human's hearing significantly, and sound with a decibel value of 20dB cannot be heard by a human, however, the current user has enabled the noise reduction function. Therefore, the tablet would display analysis result and application suggestion: the smartphone suggests the user to disable the earphone active noise reduction function, or turn up the volume level of the earphones or phone so as to ensure that after the active noise reduction function is disabled, the noise to signal ratio of the sound heard by a human remains unchanged.

Secondary, the tablet transmits instruction of disabling the active noise reduction function automatically, and turns up the volume level of the earphones.

The third scene, a laptop provides noise reduction suggestion based on the decibel value of environmental noise signal collected by the earphones, which would be described in detail as below:
After the earphone microphone collects the environmental noise signal, the environmental noise signal is input into an active noise reduction module; after processed by the module, the output decibel value of the noise signal of the current environment is 60dB.

The obtained decibel value is transmitted to the laptop via USB.

After receiving the decibel value, the laptop analyzes the decibel value, and learns that noise with a decibel value of 60dB would significantly damage a human's hearing, and the noise reduction function has been enabled by the current user. Therefore, the laptop would display analysis result and application suggestion: the laptop suggests the user to maintain the current usage state.

The laptop would not send any instruction to the earphones.

### Embodiment 6

Figure 4 is a first structural block diagram of an earphone noise reduction apparatus according to the embodiment of the present application, in which the earphone noise reduction apparatus can run in earphones. For illustration purpose, only portions relevant to the embodiment are shown.

Referring to Fig. 4, the earphone noise reduction apparatus includes:
a noise signal collection module 41 configured to utilize an earphone microphone to collect noise signal of an environment where the earphone microphone is positioned;
a noise signal transmitting module 42 configured to transmit the noise signal to a connected terminal, or transmit a noise value corresponding to the noise signal to the connected terminal;
a noise reduction function module 43 configured to receive a judgement result returned by the terminal, and enable a noise reduction function or disable the noise reduction function according to the judgement result.

In one implementation of the embodiment, the noise signal collection module 41 of the earphone noise reduction apparatus is specifically configured to utilize the earphone microphone to collect noise signal of an environment where the earphone microphone is positioned every predetermined detection interval.

In one implementation of the embodiment, the noise signal collection module 41 of the earphone noise reduction apparatus is specifically configured to utilize the earphone microphone to collect noise signal of an environment where the earphone microphone is positioned when a distance between the geographic position where earphones is currently positioned and a geographic position where the noise signal of the environment where the earphone microphone was positioned was collected last time is not less than a predetermined distance.

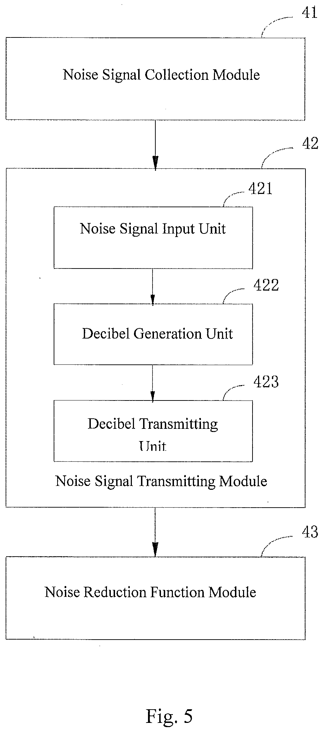

In one implementation of the embodiment, referring to Fig. 5, Figure 5 is a second structural block diagram of the earphone noise reduction apparatus according to the embodiment of the present application. The noise signal transmitting module 42 of the earphone noise reduction apparatus includes:
a noise signal input unit 421 configured to input a portion of the noise signal into a sampling channel;
a decibel generation unit 422 configured to perform analog-to-digital conversion to the portion of the noise signal in the sampling channel to generate a noise value of the noise signal;
a decibel transmitting unit 423 configured to transmit the noise value corresponding to the noise signal to a connected terminal.

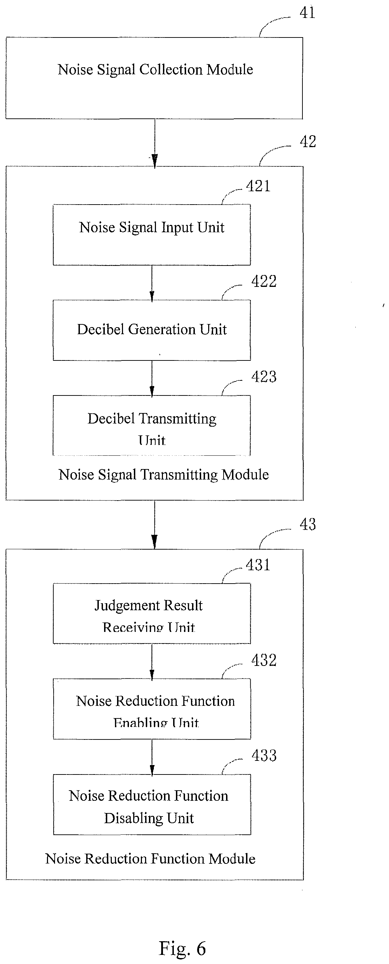

In one implementation of the embodiment, referring to Fig. 6. Figure 6 is a third structural block diagram of the earphone noise reduction apparatus according to an embodiment of the present application. The noise reduction function module 43 of the earphone noise reduction apparatus includes:
a judgement result receiving unit 431 configured to receive the judgement result returned by the terminal, the judgement result includes a judgement result of enabling noise reduction and a judgement result of disabling noise reduction;
a noise reduction function enabling unit 432 configured to enable the noise reduction function when the judgment result is the judgement result of enabling noise reduction;
a noise reduction function disabling unit 433 configured to disable the noise reduction function when the judgment result is the judgement result of disabling noise reduction.

The apparatus provided by the embodiments of the present application can be applied to corresponding process embodiments described above, which can be referred to in the description on the above embodiments and will not be further described.

It will be clear for those skilled in the art that the present application can be realized by means of software and necessary general purpose hardware on the basis of the description of the above embodiments. Mentioned program may be stored in a readable storage medium such as random access memory, flash memory, read only memory, programmable read only memory, electrically erasable programmable memory, registers, and the like. The storage medium is located in a memory, and the processor reads the information in the memory, and executes the methods described in the various embodiments of the present application in conjunction with its hardware.

The contents described above are only specific embodiments of the present application; however, the scope of the present application is not limited thereto. Those skilled in the art will readily envisage any variations or substitutions within the technical scope of the present application, which should be deemed as falling within the scope of the present application. Accordingly, the protection scope is defined by the appended claims.

## Claims

1. An earphone noise reduction method comprising:
utilizing an earphone microphone to collect noise signal of an environment where the earphone microphone is positioned when either: a distance between a geographic position where the earphone is currently positioned and a geographic position where the noise signal of the environment where the earphone microphone was positioned was collected last time is not less than a predetermined distance; or a distance between the geographic position where the earphone is currently positioned and the geographic position where the noise signal of the environment where the earphone microphone was positioned was collected last time does not exceed the predetermined distance and a time duration since the noise signal of the environment was detected last time by the earphone exceeds a predetermined time interval, wherein utilizing the earphone microphone to collect the noise signal of the environment at every predetermined time interval;
transmitting the noise signal to a terminal connected to the apparatus, or transmitting a noise value corresponding to the noise signal to the connected terminal;
receiving a judgement result returned by the terminal, and enabling a noise reduction function or disabling the noise reduction function according to the judgement result; and wherein
the transmitting a noise value corresponding to the noise signal to the connected terminal is specifically:
inputting a portion of the noise signal into a sampling channel;
performing analog-to-digital conversion to the portion of the noise signal in the sampling channel to generate a noise value of the noise signal;
transmitting the noise value corresponding to the noise signal to the connected terminal.

2. The earphone noise reduction method of claim 1, wherein after the earphone microphone collects the noise signal of the environment where the earphone microphone is positioned, the environment noise signal is divided into two paths: one path utilizes ADC conversion to perform numerical calculation so as to obtain a decibel value of the noise signal; the other path acts as analog signal to be input into an active noise reduction module.

3. The earphone noise reduction method of claim 1, wherein after the earphone microphone collects noise signal of the environment where the earphone microphone is positioned, the environmental noise signal is directly input into the active noise reduction module; calculation is performed inside the module and the decibel value of the noise signal is returned.

4. The earphone noise reduction method of claim 2 or 3, wherein the decibel value is generally transmitted to the terminal, the terminal analyzes the decibel value and returns two contents: 1) displaying an analysis result of current environmental noise signal on the terminal and providing suggestion for a user about how to use the earphones; 2) sending corresponding instruction automatically to the earphone, and adjusting the function of the earphones to a usage state which is favorite for the current noise environment.

5. The earphone noise reduction method of claim 1, wherein the receiving a judgement result returned by the terminal, and enabling a noise reduction function or disabling the noise reduction function according to the judgement result is specifically:
receiving the judgement result returned by the terminal, the judgement result includes a judgement result of enabling noise reduction function and a judgement result of disabling noise reduction function;
enabling the noise reduction function when the judgment result is enabling the noise reduction;
disabling the noise reduction function when the judgment result is disabling the noise reduction.

6. An earphone noise reduction apparatus comprising:
a noise signal collection module (41) configured to utilize an earphone microphone connected to the apparatus to collect a noise signal of an environment where the earphone microphone is positioned when either: a distance between a geographic position where the earphone is currently positioned and a geographic position where the noise signal of the environment where the earphone microphone was positioned was collected last time is not less than a predetermined distance; or a distance between the geographic position where the earphone is currently positioned and the geographic position where the noise signal of the environment where the earphone microphone was positioned was collected last time does not exceed the predetermined distance and a time duration since the noise signal of the environment was detected last time by the earphone exceeds a predetermined time interval, wherein the noise signal collecting module is specifically configured to utilize the earphone microphone to collect noise signal of the environment at every predetermined time interval;
a noise signal transmitting module (42) configured to transmit the noise signal to a terminal connected to the apparatus, or transmit a noise value corresponding to the noise signal to a terminal connected to the apparatus;
a noise reduction function module (43) configured to receive a judgement result returned by the terminal, and enable a noise reduction function or disable the noise reduction function according to the judgement result; and wherein
the noise signal transmitting module (42) comprises:
a noise signal input unit (421) configured to input a portion of the noise signal into a sampling channel;
a data generation unit (422) configured to perform analog-to-digital conversion to the portion of the noise signal in the sampling channel to generate a noise value of the noise signal;
a data transmitting unit (423) configured to transmit the noise value corresponding to the noise signal to the connected terminal.

7. The earphone noise reduction apparatus of claim 6, wherein the noise reduction function module (43) comprises:
a judgement result receiving unit (431) configured to receive the judgement result returned by the terminal, the judgement result includes a judgement result of enabling noise reduction and a judgement result of disabling noise reduction;
a noise reduction function enabling unit (432) configured to enable the noise reduction function when the judgment result is the judgement result of enabling noise reduction;
a noise reduction function disabling unit (433) configured to disable the noise reduction function when the judgment result is the judgement result of disabling noise reduction.

## Patentansprüche

1. Geräuschreduktionsverfahren für einen Kopfhörer, das die folgenden Schritte umfasst:
Verwenden eines Kopfhörermikrofons zum Sammeln eines Geräuschsignals einer Umgebung, in der das Kopfhörermikrofon positioniert ist, wenn ein Abstand zwischen einer geographischen Position, bei der der Kopfhörer gerade positioniert ist, und einer geographischen Position, bei der das Geräuschsignal der Umgebung, in der das Kopfhörermikrofon positioniert war, das letzte Mal gesammelt wurde, nicht kleiner als ein festgelegter Abstand ist; oder ein Abstand zwischen der geographischen Position, bei der der Kopfhörer gerade positioniert ist, und der geographischen Position, bei der das Geräuschsignal der Umgebung, in der das Kopfhörermikrofon positioniert war, das letzte Mal gesammelt wurde, den festgelegten Abstand nicht überschreitet, und eine Zeitspanne, seit das Geräuschsignal der Umgebung das letzte Mal durch den Kopfhörer detektiert wurde, eine festgelegte Zeitspanne überschreitet, wobei das Kopfhörermikrofon zum Sammeln des Geräuschsignals der Umgebung zu jedem festgelegten Zeitintervall verwendet wird;
Übertragen des Geräuschsignals an ein Endgerät, das mit der Vorrichtung verbunden ist, oder Übertragen eines Geräuschwerts, der dem Geräuschsignal entspricht, an das verbundene Endgerät;
Empfangen eines Bewertungsergebnisses, das von dem Endgerät zurückgegeben wird, und Einschalten einer Geräuschreduktionsfunktion oder Ausschalten der Geräuschreduktionsfunktion entsprechend dem Bewertungsergebnis; wobei
das Übertragen eines Geräuschwerts entsprechend dem Geräuschsignal an das verbundene Endgerät insbesondere die folgenden Schritte umfasst:
Eingeben eines Teils des Geräuschsignals in einen Abtastkanal;
Durchführen einer Analog/Digital-Umsetzung des Teils des Geräuschsignals in dem Abtastkanal zum Erzeugen eines Geräuschwerts des Geräuschsignals;
Übertragen des Geräuschwerts entsprechend dem Geräuschsignal an das verbundene Endgerät.

2. Geräuschreduktionsverfahren für einen Kopfhörer nach Anspruch 1, wobei dann, wenn das Kopfhörermikrofon das Geräuschsignal der Umgebung gesammelt hat, in der das Kopfhörermikrofon positioniert ist, das Umgebungsgeräuschsignal in zwei Pfade unterteilt wird, wovon ein Pfad die ADC-Umsetzung nutzt, um eine numerische Berechnung durchzuführen, um so einen Dezibelwert des Geräuschsignals zu erhalten; und wovon der andere Pfad als ein analoges Signal dient, das in ein Modul zur aktiven Geräuschreduktion eingegeben wird.

3. Geräuschreduktionsverfahren für einen Kopfhörer nach Anspruch 1, wobei dann, wenn das Kopfhörermikrofon das Geräuschsignal der Umgebung, in der das Kopfhörermikrofon positioniert ist, gesammelt hat, das Umgebungsgeräuschsignal direkt in das Modul zur aktiven Geräuschreduktion eingegeben wird; wobei eine Berechnung im Inneren des Moduls durchgeführt wird und der Dezibelwert des Geräuschsignals zurückgegeben wird.

4. Geräuschreduktionsverfahren für einen Kopfhörer nach Anspruch 2 oder 3, wobei der Dezibelwert im Allgemeinen an das Endgerät übertragen wird, wobei das Endgerät den Dezibelwert analysiert und zwei Inhalte zurückgibt: 1) Anzeigen eines Analyseergebnisses eines aktuellen Umgebungsgeräuschsignals auf dem Endgerät und Bereitstellen eines Vorschlags für einen Benutzer, wie die Kopfhörer zu verwenden sind; 2) Übertragen einer entsprechenden Anweisung automatisch an den Kopfhörer und Einstellen der Funktion des Kopfhörers in einen Nutzungszustand, der für die aktuelle Geräuschumgebung am besten geeignet ist.

5. Geräuschreduktionsverfahren für einen Kopfhörer nach Anspruch 1, wobei das Empfangen eines Bewertungsergebnisses, das von dem Endgerät zurückgegeben wird, und das Einschalten einer Geräuschreduktionsfunktion oder das Ausschalten der Geräuschreduktionsfunktion entsprechend dem Bewertungsergebnis die folgenden Schritte umfasst:
Empfangen des Bewertungsergebnisses, das durch das Endgerät zurückgegeben wird, wobei das Bewertungsergebnis ein Bewertungsergebnis zum Einschalten der Geräuschreduktionsfunktion und ein Bewertungsergebnis zum Ausschalten der Geräuschreduktionsfunktion umfasst;
Einschalten der Geräuschreduktionsfunktion, wenn das Bewertungsergebnis die Geräuschreduktion einschaltet;
Ausschalten der Geräuschreduktionsfunktion, wenn das Bewertungsergebnis die Geräuschreduktion ausschaltet.

6. Geräuschreduktionsvorrichtung für einen Kopfhörer, die Folgendes umfasst:
ein Modul (41) zum Sammeln eines Geräuschsignals, das konfiguriert ist, ein Kopfhörermikrofon zu verwenden, das mit der Vorrichtung verbunden ist, um ein Geräuschsignal einer Umgebung zu sammeln, in der das Kopfhörermikrofon positioniert ist, wenn ein Abstand zwischen einer geographischen Position, bei der der Kopfhörer gerade positioniert ist, und einer geographischen Position, bei der das Geräuschsignal der Umgebung, in der das Kopfhörermikrofon positioniert war, das letzte Mal gesammelt wurde, nicht kleiner als ein festgelegter Abstand ist; oder wenn ein Abstand zwischen der geographischen Position, bei der der Kopfhörer gerade positioniert ist, und der geographischen Position, bei der das Geräuschsignal der Umgebung, in der das Kopfhörermikrofon positioniert war, das letzte Mal gesammelt wurde, den festgelegten Abstand nicht überschreitet, und eine Zeitspanne, seit das Geräuschsignal der Umgebung das letzte Mal durch den Kopfhörer detektiert wurde, eine festgelegte Zeitspanne überschreitet, wobei das Modul zum Sammeln des Geräuschsignals insbesondere konfiguriert ist, das Kopfhörermikrofon zu verwenden, um ein Geräuschsignal der Umgebung zu jedem festgelegten Zeitintervall zu sammeln;
ein Modul (42) zum Übertragen eines Geräuschsignals, das konfiguriert ist, das Geräuschsignal an ein Endgerät zu übertragen, das mit der Vorrichtung verbunden ist, oder einen Geräuschwert entsprechend dem Geräuschsignal an ein Endgerät zu übertragen, das mit der Vorrichtung verbunden ist;
ein Modul (43) mit Geräuschreduktionsfunktion, das konfiguriert ist, ein Bewertungsergebnis zu erhalten, das von dem Endgerät zurückgegeben wird, und entsprechend dem Bewertungsergebnis eine Geräuschreduktionsfunktion einzuschalten oder die Geräuschreduktion auszuschalten;
und wobei das Modul (42) zum Übertragen eines Geräuschsignals Folgendes umfasst:
eine Geräuschsignal-Eingabeeinheit (421), die konfiguriert ist, einen Teil des Geräuschsignals in einen Abtastkanal einzugeben;
eine Datenerzeugungseinheit (422), die konfiguriert ist, eine Analog/DigitalUmsetzung des Teils des Geräuschsignals in dem Abtastkanal durchzuführen, um einen Geräuschwert des Geräuschsignals zu erzeugen;
eine Datenübertragungseinheit (423), die konfiguriert ist, den Geräuschwert entsprechend dem Geräuschsignal an das verbundene Endgerät zu übertragen.

7. Vorrichtung zur Geräuschreduktion in einem Kopfhörer nach Anspruch 6, wobei das Modul (43) mit Geräuschreduktionsfunktion Folgendes umfasst:
eine Bewertungsergebnis-Empfangseinheit (431), die konfiguriert ist, das Bewertungsergebnis zu empfangen, das von dem Endgerät zurückgegeben wird, wobei das Bewertungsergebnis ein Bewertungsergebnis zum Einschalten der Geräuschreduktion und ein Bewertungsergebnis zum Ausschalten der Geräuschreduktion umfasst;
eine Einheit (432) zum Einschalten der Geräuschreduktionsfunktion, die konfiguriert ist, die Geräuschreduktionsfunktion einzuschalten, wenn das Bewertungsergebnis das Bewertungsergebnis zum Einschalten der Geräuschreduktion ist;
eine Einheit (433) zum Ausschalten der Geräuschreduktionsfunktion, die konfiguriert ist, die Geräuschreduktionsfunktion auszuschalten, wenn das Bewertungsergebnis das Bewertungsergebnis zum Ausschalten der Geräuschreduktion ist.

## Revendications

1. Procédé de réduction de bruit dans un écouteur, comprenant les étapes consistant à :
utiliser un microphone d'écouteur pour collecter un signal sonore d'un environnement dans lequel le microphone d'écouteur est positionné quand : soit une distance entre une position géographique à laquelle l'écouteur est actuellement positionné et une position géographique à laquelle le signal sonore de l'environnement dans lequel le microphone écouteur était positionné a été collecté la dernière fois n'est pas inférieure à une distance prédéterminée ; soit une distance entre la position géographique à laquelle l'écouteur est actuellement positionné et la position géographique à laquelle le signal sonore de l'environnement dans lequel le microphone d'écouteur était positionné a été collecté la dernière fois n'excède pas la distance prédéterminée et une durée depuis que le signal sonore de l'environnement a été détecté la dernière fois par l'écouteur dépasse un intervalle de temps prédéterminé, dans lequel le microphone d'écouteur est utilisé pour collecter le signal sonore de l'environnement à chaque intervalle de temps prédéterminé ;
transmettre le signal sonore à un terminal connecté à l'appareil, ou transmettre une valeur sonore correspondant au signal sonore au terminal connecté ;
recevoir un résultat de jugement renvoyé par le terminal, et activer une fonction de réduction de bruit ou désactiver la fonction de réduction de bruit en accord avec le résultat de jugement ; et dans lequel l'étape de transmission d'une valeur sonore correspondant au signal sonore au terminal connecté consiste spécifiquement à :
entrer une portion du signal sonore jusque dans un canal d'échantillonnage ;
exécuter une conversion analogique/numérique sur la portion du signal sonore dans le canal d'échantillonnage pour générer une valeur sonore du signal sonore ;
transmettre la valeur sonore correspondant au signal sonore au terminal connecté.

2. Procédé de réduction de bruit dans un écouteur selon la revendication 1, dans lequel,
après que le microphone d'écouteur a collecté le signal sonore de l'environnement dans lequel le microphone d'écouteur est positionné, le signal sonore d'environnement est divisé en deux chemins : un chemin utilisant une conversion de convertisseur A/N pour exécuter un calcul numérique de manière à obtenir une valeur en décibels du signal sonore ; l'autre chemin agissant à titre de signal analogique qu'il s'agit d'entrer jusque dans un module de réduction active de bruit.

3. Procédé de réduction de bruit dans un écouteur selon la revendication 1, dans lequel,
après que le microphone d'écouteur a collecté le signal sonore de l'environnement dans lequel le microphone d'écouteur est positionné, le signal sonore d'environnement est directement entré jusque dans le module de réduction active de bruit ; un calcul est exécuté à l'intérieur du module et la valeur en décibels du signal sonore est renvoyée.

4. Procédé de réduction de bruit dans un écouteur selon la revendication 2 ou 3, dans lequel
la valeur en décibels est généralement transmise au terminal, le terminal analyse la valeur en décibels et renvoie deux contenus : 1) afficher un résultat d'analyse du signal sonore d'environnement actuel sur le terminal et fournir une suggestion pour un utilisateur quant à savoir comment utiliser les écouteurs ; 2) envoyer une instruction correspondante automatiquement à l'écouteur, et ajuster la fonction des écouteurs à un état d'usage qui est favori pour l'environnement de bruit actuel.

5. Procédé de réduction de bruit dans un écouteur selon la revendication 1, dans lequel
la réception d'un résultat du jugement renvoyé par le terminal, et l'activation d'une fonction de réduction de bruit ou la désactivation de la fonction de réduction de bruit selon le résultat de jugement consiste spécifiquement à :
recevoir le résultat de jugement renvoyé par le terminal, le résultat de jugement incluant un résultat de jugement consistant à activer une fonction de réduction de bruit et un résultat de jugement consistant à désactiver une fonction de réduction de bruit ;
activer la fonction de réduction de bruit quand le résultat de jugement est d'activer la réduction de bruit ;
désactiver la fonction de réduction de bruit quand le résultat de jugement est de désactiver la réduction de bruit.

6. Appareil de réduction de bruit dans un écouteur comprenant :
un module de collecte de signal sonore (41) configuré pour utiliser un microphone d'écouteur connecté à l'appareil pour collecter un signal sonore d'un environnement dans lequel le microphone d'écouteur est positionné quand : soit une distance entre une position géographique à laquelle l'écouteur est actuellement positionné et une position géographique à laquelle le signal sonore de l'environnement dans lequel le microphone écouteur était positionné a été collecté la dernière fois n'est pas inférieure à une distance prédéterminée ; soit une distance entre la position géographique à laquelle l'écouteur est actuellement positionné et la position géographique à laquelle le signal sonore de l'environnement dans lequel le microphone d'écouteur était positionné a été collecté la dernière fois n'excède pas la distance prédéterminée et une durée depuis que le signal sonore de l'environnement a été détecté la dernière fois par l'écouteur dépasse un intervalle de temps prédéterminé, dans lequel le module de collecte de signal sonore est spécifiquement configuré pour utiliser le microphone d'écouteur pour collecter un signal sonore de l'environnement à chaque intervalle de temps prédéterminé ;
un module de transmission de signal sonore (42) configuré pour transmettre le signal sonore à un terminal connecté à l'appareil, ou pour transmettre une valeur sonore correspondant au signal sonore à un terminal connecté à l'appareil ;
un module de fonction de réduction de bruit (43) configuré pour recevoir un résultat de jugement renvoyé par le terminal, et pour activer une fonction de réduction de bruit ou désactiver la fonction de réduction de bruit en accord avec le résultat de jugement ; et dans lequel
le module de transmission de signal sonore (42) comprend :
une unité d'entrée de signal sonore (421) configurée pour entrer une portion du signal sonore jusque dans un canal d'échantillonnage ;
une unité de génération de données (422) configurée pour exécuter une conversion analogique/numérique sur la portion du signal sonore dans le canal d'échantillonnage pour générer une valeur sonore du signal sonore ;
une unité de transmission de données (423) configurée pour transmettre la valeur sonore correspondant au signal sonore au terminal connecté.

7. Appareil de réduction de bruit dans un écouteur selon la revendication 6, dans lequel
le module de fonction de réduction de bruit (43) comprend :
une unité de réception de résultat de jugement (431) configurée pour recevoir le résultat de jugement renvoyé par le terminal, le résultat de jugement incluant un résultat de jugement consistant à activer une réduction de bruit et un résultat de jugement consistant à désactiver une réduction de bruit ;
une unité d'activation de fonction de réduction de bruit (432) configurée pour activer la fonction de réduction de bruit quand le résultat de jugement est le résultat de jugement consistant à activer la réduction de bruit ;
une unité de désactivation de fonction de réduction de bruit (433) configurée pour désactiver la fonction de réduction de bruit quand le résultat de jugement est le résultat de jugement consistant à désactiver la réduction de bruit.
